# FASCICULE DE BREVET EUROPEEN

(11) **EP 1 468 993 B1**
(45) Date de publication et mention de la délivrance du brevet: **21.12.2005**
(21) Numéro de dépôt: 04290935.8
(22) Date de dépôt: 08.04.2004
(51) Int. Cl.: C07D 231/04

(54) **Procédé de préparation de dérivés de l'acide hexahydropyridazine-3-carboxylique**
Verfahren zur Herstellung von Hexahydropyridazin-3-carbonsäure-Derivaten
Process for the preparation of hexahydropyridazine-3-carboxylic acid derivatives

(30) Priorité: 16.04.2003 FR 0304763
(43) Date de publication de la demande: 20.10.2004
(73) Titulaire: ISOCHEM, 75004 Paris (FR)
(72) Inventeur: Lhermitter, Hervé, 75019 Paris (FR); Vincent, Charles-Henri, 60460 Precy sur Oise (FR); Picherit, Christian, 95570 Boussemont (FR)
(74) Mandataire: Ahner, Francis

(56) Documents cités:
- WO-A-99/55724
- US-B1- 6 177 565
- US-B1- 6 201 118
- TOYA T. ET AL: "Cyclic dibenzoylhydrazines reproducing the conformation of Ecdysone agonists, RH-5849" BIOORGANIC & MED. CHEM., vol. 10, 2002, pages 953-961, XP002257781
- HINMAN R. L. ET AL.: "Alkylation of some diacylhadrazines" J. ORG. CHEM., vol. 24, 1959, pages 724-725, XP002257782

## Description

La présente invention concerne un procédé amélioré de préparation de dérivés de l'acide hexahydropyridazine-3-carboxylique.

L'acide hexahydropyridazine-3-carboxylique et ses dérivés sont des composés très utiles comme intermédiaires pour la préparation de médicaments.

Plusieurs procédés ont été proposés pour leur synthèse. Dans la demande de brevet WO 99/55724, il est décrit un procédé de préparation d'acide hexahydropyridazine-3-carboxylique portant en position 1 un substituant arylméthyloxycarbonyle à partir de 2,5-dihalogénopentanoate d'alkyle et de 1,2-hydrazinedicarboxylate de bisarylméthyle.

Le procédé se déroule en 2 étapes. Dans la première étape, on forme d'abord le composé intermédiaire tétrahydro-1,2-bisarylméthyle qui est isolé puis il est soumis à un traitement basique pour obtenir le dérivé de l'acide souhaité.

Le schéma réactionnel est le suivant :

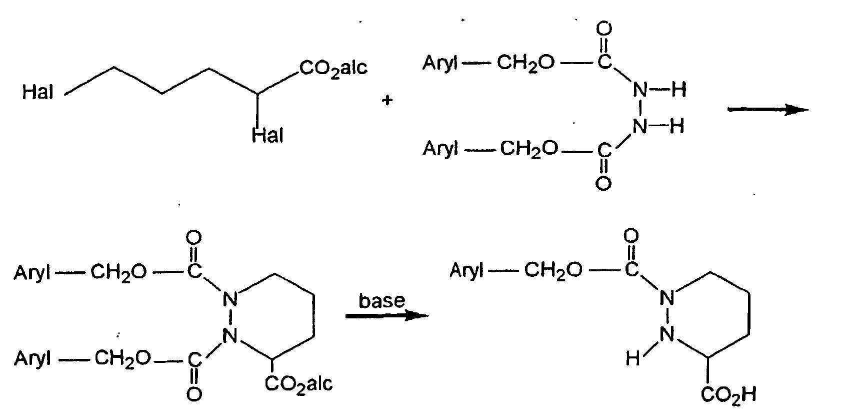

La mise en oeuvre de ce procédé présente des inconvénients.

De très grandes quantités de solvants sont utilisées. Certains de ces solvants sont cancérigènes tel que le diglyme, solvant de la première étape. D'autres sont facilement inflammables tels que les éthers, comme l'éther isopropylique, qui servent à isoler les composés. D'autres sont nocifs pour la santé des opérateurs et pour l'environnement tels que l'éthanol, solvant de la réaction de la deuxième étape ou les solvants chlorés, comme le dichlorométhane, employés pour les extractions des composés.

Ces grandes quantités de solvant nécessitent l'utilisation de réacteurs volumineux, entraînent des manipulations nombreuses pour les éliminer ou effectuer des échanges. Les temps de main-d'oeuvre, d'occupation des réacteurs et des autres installations sont donc très importants. Il en résulte aussi des pertes de rendement et une pureté des composés non satisfaisante. De plus selon ce procédé, certaines purifications des composés s'effectuent par chromatographie sur support solide qui est une méthode non rentable industriellement.

Il existait par conséquent un besoin d'améliorer les conditions de mise en oeuvre du procédé afin d'obtenir plus rapidement et plus économiquement les dérivés souhaités.

L'invention a pour objet un procédé de préparation des dérivés de l'acide hexahydropyridazine-3-carboxylique de formule (I) 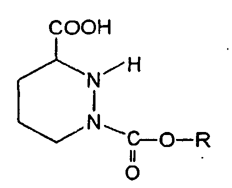 dans laquelle R représente un radical alkyle saturé ou insaturé, substitué ou non, un radical aralkyle substitué ou non ou un radical aryle substitué ou non, caractérisé en ce que l'on fait réagir un composé de formule (II) 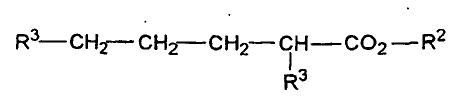 dans laquelle R² représente un radical alkyle substitué ou non, et
R³ représente un atome d'halogène ou un groupe organique nucléofuge,
avec un composé de formule (III) 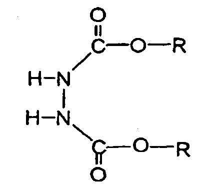 dans laquelle R a la signification précédente,
en présence d'une base dont le pK est égal ou supérieur à 8,5, dans un solvant organique choisi parmi les cétones, pour obtenir le composé intermédiaire tétrahydro-1,2,3-pyridazine tricarboxylate de formule (IV) 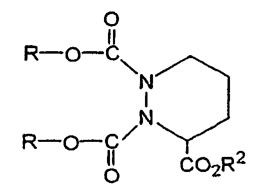 dans laquelle R et R² ont les significations précédentes, qui n'est pas isolé et que l'on traite par un milieu aqueux basique, pour obtenir les dérivés de l'acide hexahydropyridazine-3-carboxylique de formule (I).

Selon ce procédé, les quantités de solvant organiques utilisées sont très réduites. Seule la première réaction est effectuée en présence d'un solvant organique de type cétonique, par conséquent non toxique.

Le composé intermédiaire de formule (IV) tétrahydro-1,2,3-pyridazine-tricarboxylate n'est pas isolé du milieu. Par conséquent, aucun traitement avec un acide, aucune extraction avec un solvant organique comme effectués dans l'exemple 1d) page 9 de la demande de brevet WO 99/55724 n'est nécessaire.

Le solvant pour effectuer la deuxième réaction ne pose pas de problème puisqu'il s'agit de l'eau. On évite ainsi l'utilisation d'un solvant alcoolique qu'il faut ensuite éliminer comme c'est le cas dans l'exemple 1, paragraphe e) pages 9 et 10 du document de l'art antérieur précédemment cité.

La productivité selon le procédé de l'invention est ainsi considérablement augmentée.

Par ailleurs, la récupération du dérivé final de formule (I) s'effectue simplement par cristallisation dans un milieu biphasique eau/solvant. On évite de cette façon les nombreuses extractions au dichlorométhane pratiquées selon le procédé antérieur.

Le procédé selon l'invention permet d'obtenir les dérivés de formule (I) aussi bien sous leur forme racémique que sous leur forme optiquement active R ou S.

Les composés de formule (II) et (III) utilisés comme produits de départ sont des composés connus qui se trouvent dans le commerce ou qui peuvent se préparer selon des méthodes connues.

Dans les composés de formule (I) et (III), R lorsqu'il représente un radical alkyle est en particulier un radical en C₁ à C₈. Il peut être saturé ou insaturé comme par exemple le radical allyle ou vinyle. Comme substituants, on peut citer notamment les atomes d'halogène tel que le chlore, de préférence non situés en position 1.

Lorsque R représente un radical aralkyle, il est de préférence un radical de formule -CH₂-R¹ dans laquelle R¹ est un radical aryle substitué ou non.

Le radical R¹ peut porter un ou plusieurs substituants choisis notamment parmi les atomes d'halogène, tel que de chlore, les radicaux alkyle en particulier en C₁ à C₃.

R peut également représenter un radical aryle portant ou non un ou plusieurs substituants choisis notamment parmi les radicaux alkyle, en particulier en C₁ à C₄, les radicaux alcoxy, en particulier en C₁ à C₄, et phénoxy, les atomes d'halogène, tel que de chlore et de fluor.

Par radical aryle, on entend un radical aromatique hydrocarboné mono ou polycyclique, les cycles étant condensés ou non, en particulier un radical en C₆ à C₁₄ tel que le radical naphtyle ou biphényle, de préférence le radical phényle.

R² peut représenter un radical alkyle linéaire ou ramifié, substitué ou non, notamment en C₁ à C₂₀, en particulier en C₁ à C₈, de préférence en C₁ à C₄. Comme substituants de R², on peut notamment citer les radicaux alcoxy en particulier méthoxy et éthoxy.

Comme exemple de radical R², on peut citer les radicaux isopropyle et isobutyle. Les radicaux méthyle et éthyle conviennent bien.

R³ représente un atome d'halogène ou un groupe organique qui peut être séparé facilement du reste de la molécule dans les conditions du procédé, tel qu'un groupe mésylate ou tosylate.

Comme atomes d'halogène représentés par R³, on peut citer les atomes de chlore, de brome ou d'iode et de préférence les atomes de chlore ou de brome.

On a trouvé qu'une amélioration du procédé antérieur consiste à faire réagir le composé de formule (II) avec le composé de formule (III) dans un milieu solvant organique choisi parmi les cétones. Comme cétones, on peut citer la méthyléthylcétone, la méthyl isobutylcétone, la méthyltertiobutylcétone, la diisopropylcétone, utilisées seules ou en mélange.

L'acétone est le solvant préféré.

Le composé (II) est généralement utilisé en quantité comprise entre 1 et 1,5, de préférence entre 1 et 1,1 mole par mole de composé de formule (III).

La réaction a lieu en présence d'une base dont le pK est supérieur ou égal à 8,5. Comme bases, on peut citer les carbonates de métaux alcalins, en particulier comme le sodium ou le potassium, les amines tertiaires comme la triéthylamine, la N-méthylmorpholine. La base est de préférence présente dans le milieu en quantité comprise entre 2 et 3 moles par mole du composé de formule (II).

On peut pour faciliter la réaction ajouter dans le milieu réactionnel, un catalyseur de transfert de phase solide-liquide tel qu'un sel de tétraalkylammonium, ou un tensio-actif.

La température de la réaction dépend du solvant utilisé. Elle est généralement comprise entre 50° et 100°C. De préférence, on choisit une température comprise entre 55° et 65°C. La durée de la réaction est également fonction du solvant utilisé. Elle est généralement d'environ 25 à 35 heures.

Contrairement au procédé selon l'art antérieur, le dérivé d'hydrazine intermédiaire formé, de formule (IV), n'est pas isolé. On a trouvé que l'on pouvait le faire réagir avantageusement dans de nouvelles conditions sans que son isolement soit nécessaire. On évite ainsi les nombreux traitements et extractions obligatoires lorsqu'on veut le récupérer et les pertes de produits qui en résultent.

Le composé de formule (IV) est ainsi traité par un milieu aqueux basique. De préférence, le milieu aqueux doit être fortement basique, en particulier le pH doit être supérieur ou égal à 12.

La basicité est apportée par un composé minéral ou organique. Une base minérale choisie parmi les hydroxydes de métaux alcalins, en particulier de sodium et de potassium, convient bien. Comme composés organiques apportant une forte basicité, on peut citer les alcoolates alcalins ou alcalino-terreux comme le méthylate, l'éthylate ou le tertiobutylate de sodium ou de potassium.

La quantité de base à employer est généralement comprise entre 3 et 6 équivalents molaires par rapport au composé (III). Une quantité de 4 à 5 équivalents molaires convient bien. La quantité de base choisie peut être apportée en une ou plusieurs fractions.

De préférence, pour des raisons pratiques les hydroxydes de métaux alcalins sont utilisés en solution aqueuse.

On a trouvé qu'il était beaucoup plus intéressant d'effectuer la deuxième réaction en utilisant l'eau comme solvants.

La quantité d'eau employée est généralement comprise entre 1 et 10 litres par kilogramme de composé (III).

On élimine généralement la majorité du solvant cétonique utilisé pour la première réaction, par exemple par distillation, avant d'effectuer la mise en oeuvre de la deuxième réaction. De préférence la totalité du solvant est enlevée.

La température de la deuxième réaction est généralement comprise entre 20°C et 60°C, de préférence entre 25°C et 55°C. Différents paliers successifs de température peuvent être effectués dans ces gammes. La durée de la réaction est en général de 2 à 12 heures.

On a trouvé également que l'on peut récupérer très facilement le composé de formule (I) en le faisant cristalliser directement dans le milieu réactionnel et cela malgré la présence de l'alcool, en particulier aralkylique, formé au cours de la dernière réaction. Pour ce faire, on mélange le milieu réactionnel avec un acide et un solvant organique dans lequel le composé (I) n'est pas soluble. De préférence, le solvant est utilisé avant l'acide.

Comme acides, on peut citer l'acide acétique ou l'acide formique et de préférence l'acide chlorhydrique ou l'acide sulfurique.

On emploie l'acide en quantité suffisante pour amener le pH du milieu entre 0, 5 et 2 et de préférence aux environs de 1. L'acide chlorhydrique convient bien.

Comme solvants ne dissolvant pas les composés de formule (I), on peut en particulier citer les solvants diluant les alcools, solvants en général peu polaires tels que les hydrocarbures aromatiques, les hydrocarbures aliphatiques, de préférence non chlorés, les éthers et les acétates.

De façon préférée, on utilise le toluène et les xylènes.

Il n'est pas nécessaire d'employer une grande quantité de solvant organique. Généralement, cette quantité est comprise entre 1 et 5 litres par kilogramme de composé (III).

Les composés de formule (I) sont alors en suspension dans la phase organique. Cette phase peut être séparée de la phase aqueuse selon des méthodes classiques, par exemple par décantation et les composés facilement récupérés notamment par filtration.

Le procédé selon l'invention permet d'obtenir les dérivés de formule (I) avec de bons rendements, souvent supérieurs ou égaux à 70 % et une grande pureté déterminée par HPLC supérieure à 97 %.

L'exemple qui suit illustre l'invention sans toutefois la limiter.

### EXEMPLE 1

Dans un réacteur de 8 litres équipé, on introduit 1 kg (3,3 mol) de 1,2-dibenzyloxycarbonylhydrazine, 0,025 kg de bromure de tétrabutylammonium, 1 kg (2,2 éq) de carbonate de potassium (K₂CO₃) en poudre fine et 5 1 d'acétone et on agite le mélange.

On ajoute ensuite 1 kg (3,65 mol) de 2,5-dibromovalérate de méthyle et on chauffe au reflux de l'acétone pendant 24 heures.

On refroidit le mélange à 0°C. On sépare les solides par essorage.

On concentre ensuite le milieu liquide sous pression réduite jusqu'à l'obtention d'un concentrat huileux.

On ajoute alors 1 l d'eau puis 1,66 l de lessive de soude à 30 % en maintenant la température à 40° et on agite le mélange réactionnel pendant 5 à 7 heures à cette température.

On ajoute 2,6 l de toluène puis lentement 1,4 l d'acide chlorhydrique à 36 % pour obtenir un pH d'environ 1.

L'acide 1-benzyloxycarbonylhexahydropyridazin-3-yl carboxylique de formule (I) cristallise.

On l'essore, on le lave avec du toluène puis avec de l'eau, et on le sèche. On recueille ainsi 0,62 kg d'acide sec (rendement global 71 %) de pureté, déterminée par HPLC, supérieure à 97,0 %.

## Revendications

1. Procédé de préparation des dérivés de l'acide hexahydropyridazine-3-carboxylique de formule (I) 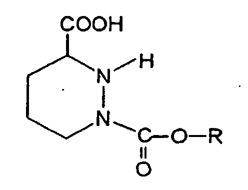 dans laquelle R représente un radical alkyle saturé ou insaturé, substitué ou non, un radical aralkyle substitué ou non ou un radical aryle substitué ou non, **caractérisé en ce que** l'on fait réagir un composé de formule (II) 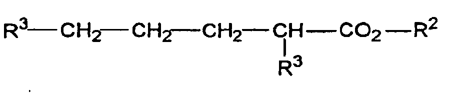 dans laquelle R² représente un radical alkyle substitué ou non, et
R³ représente un atome d'halogène ou un groupe organique nucléofuge,
avec un composé de formule (III) 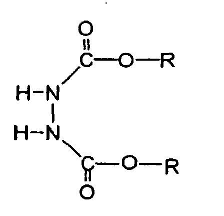 dans laquelle R a la signification précédente,
en présence d'une base dont le pK est égal ou supérieur à 8,5, dans un solvant organique choisi parmi les cétones, pour obtenir le composé intermédiaire tétrahydro-1,2,3-pyridazine tricarboxylate de formule (IV) 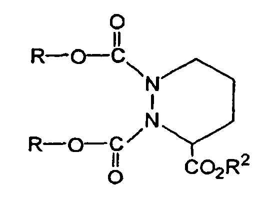 dans laquelle R et R² ont les significations précédentes, qui n'est pas isolé et que l'on traite par un milieu aqueux basique pour obtenir le dérivé de l'acide hexahydropyridazine-3-carboxylique de formule (I).

2. Procédé selon la revendication 1, **caractérisé en ce que** le solvant organique est choisi parmi l'acétone, la méthyléthylcétone, la méthylisobutylcétone, la méthyltertiobutylcétone, la diisopropylcétone et leurs mélanges.

3. Procédé selon la revendication 1 ou 2, **caractérisé en ce que** la base utilisée dans la première réaction est choisie dans le groupe constitué par les carbonates de métaux alcalins et les amines tertiaires.

4. Procédé selon l'une quelconque des revendications précédentes, **caractérisé en ce que** le solvant est l'acétone.

5. Procédé selon l'une quelconque des revendications précédentes, **caractérisé en ce que** la base utilisée dans la première réaction est le carbonate de potassium.

6. Procédé selon l'une quelconque des revendications précédentes, **caractérisé en ce que** la base utilisée pour la deuxième réaction est choisie parmi les hydroxydes de métaux alcalins et les alcoolates de métaux alcalins ou alcalino-terreux.

7. Procédé selon la revendication précédente, **caractérisé en ce que** les hydroxydes de métaux alcalins sont utilisés en solution aqueuse.

8. Procédé selon la revendication 6 ou 7, **caractérisé en ce que** la base minérale est la soude ou la potasse.

9. Procédé selon l'une quelconque des revendications précédentes, **caractérisé en ce que** pour la deuxième réaction, la température est comprise entre 25°C et 55°C et le volume d'eau est compris entre 1 et 10 litres par kilogramme de composé de formule (III).

10. Procédé selon la revendication précédente **caractérisé en ce que** la réaction est réalisée en effectuant différents paliers successifs de température compris dans la gamme.

11. Procédé selon l'une quelconque des revendications précédentes, **caractérisé en ce qu'**on obtient le composé de formule (I) sous forme cristalline en mélangeant le milieu réactionnel avec un solvant dans lequel le composé de formule (I) n'est pas soluble et diluant les alcools et en amenant le pH du milieu à une valeur comprise entre 0,5 et 2 au moyen d'un acide.

12. Procédé selon la revendication précédente, **caractérisé en ce que** le solvant est choisi dans le groupe constitué par les hydrocarbures aromatiques, les hydrocarbures aliphatiques, les éthers et les acétates.

13. Procédé selon la revendication 11 ou 12, **caractérisé en ce que** l'acide est l'acide chlorhydrique.

14. Procédé selon l'une quelconque des revendications précédentes, **caractérisé en ce que** R représente le radical -CH₂-R¹, R¹ représente le radical phényle ou naphtyle, R² représente un radical alkyle en C₁ à C₄ et R³ représente un atome d'halogène.

15. Procédé selon la revendication précédente **caractérisé en ce que** R¹ représente le radical phényle, R² représente le radical méthyle et R³ représente un atome de brome.

## Patentansprüche

1. Verfahren zur Herstellung von Hexahydropyridazin-3-carbonsäure-Derivaten der Formel (I) 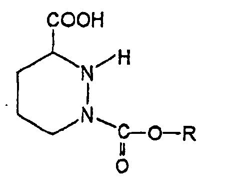 in der R einen gesättigten oder ungesättigten, substituierten oder unsubstituierten Alkylrest, einen substituierten oder unsubstituierten Aralkylrest oder einen substituierten oder unsubstituierten Arylrest darstellt, **dadurch gekennzeichnet, dass** man eine Verbindung der Formel (II) 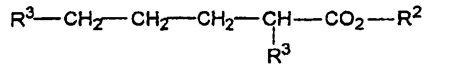 in der R² einen substituierten oder unsubstituierten Alkylrest darstellt und R³ ein Halogenatom oder eine nukleofuge organische Gruppe darstellt, mit einer Verbindung der Formel (III) 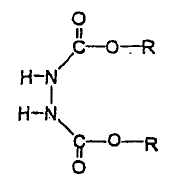 in der R die vorstehende Bedeutung aufweist,
in Anwesenheit einer Base, deren pK gleich oder größer als 8,5 ist, in einem organischen Lösungsmittel, das aus Ketonen ausgewählt ist, reagieren lässt, um die Zwischenprodukt-Verbindung Tetrahydro-1,2,3-pyridazintricarboxylat der Formel (IV) 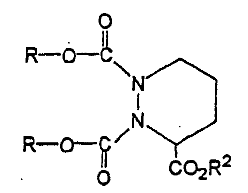 zu erhalten, in der R und R² die vorstehenden Bedeutungen aufweisen, welche nicht isoliert wird und die man mit einem wässrigen basischen Medium behandelt, um das Hexahydropyridazin-3-carbonsäure-Derivat der Formel (I) zu erhalten.

2. Verfahren nach Anspruch 1, **dadurch gekennzeichnet, dass** das organische Lösungsmittel aus Aceton, Methylethylketon, Methylisobutylketon, Methyltertiärbutylketon, Diisopropylketon und deren Mischungen ausgewählt ist.

3. Verfahren nach Anspruch 1 oder 2, **dadurch gekennzeichnet, dass** die in der ersten Reaktion verwendete Base aus der Gruppe ausgewählt ist, die aus Alkalimetallcarbonaten und tertiären Aminen besteht.

4. Verfahren nach irgendeinem der vorangehenden Ansprüche, **dadurch gekennzeichnet, dass** das Lösungsmittel Aceton ist.

5. Verfahren nach irgendeinem der vorangehenden Ansprüche, **dadurch gekennzeichnet, dass** die in der ersten Reaktion verwendete Base Kaliumcarbonat ist.

6. Verfahren nach irgendeinem der vorangehenden Ansprüche, **dadurch gekennzeichnet, dass** die für die zweite Reaktion verwendete Base aus Alkalimetallhydroxiden und Alkali- oder Erdalkalimetallalkoholaten ausgewählt ist.

7. Verfahren nach dem vorangehenden Anspruch, **dadurch gekennzeichnet, dass** die Alkalimetallhydroxide in wässriger Lösung verwendet werden.

8. Verfahren nach Anspruch 6 oder 7, **dadurch gekennzeichnet, dass** die Mineralbase Natriumhydroxid oder Kaliumhydroxid ist.

9. Verfahren nach irgendeinem der vorangehenden Ansprüche, **dadurch gekennzeichnet, dass** bei der zweiten Reaktion die Temperatur zwischen 25°C und 55°C einschließlich liegt und das Wasservolumen zwischen 1 und 10 Litern einschließlich pro Kilogramm Verbindung der Formel (III) liegt.

10. Verfahren nach dem vorangehenden Anspruch, **dadurch gekennzeichnet, dass** die Reaktion durchgeführt wird, indem man verschiedene aufeinanderfolgende Temperaturplateaus bewirkt, die in dem Bereich eingeschlossen sind.

11. Verfahren nach irgendeinem der vorangehenden Ansprüche, **dadurch gekennzeichnet, dass** man die Verbindung der Formel (I) in kristalliner Form erhält, indem man das Reaktionsmedium mit einem Lösungsmittel mischt, in dem die Verbindung der Formel (I) nicht löslich ist, und die Alkohole verdünnt und den pH des Mediums mittels einer Säure auf einen Wert zwischen 0,5 und 2 einschließlich bringt.

12. Verfahren nach dem vorangehenden Anspruch, **dadurch gekennzeichnet, dass** das Lösungsmittel ausgewählt ist aus der Gruppe bestehend aus aromatischen Kohlenwasserstoffen, aliphatischen Kohlenwasserstoffen, Ethern und Acetaten.

13. Verfahren nach Anspruch 11 oder 12, **dadurch gekennzeichnet, dass** die Säure Chlorwasserstoffsäure ist.

14. Verfahren nach irgendeinem der vorangehenden Ansprüche, **dadurch gekennzeichnet, dass** R den Rest -CH₂-R¹ darstellt, R¹ einen Phenyl- oder Naphthylrest darstellt, R² einen C₁- bis C₄-Alkylrest darstellt und R³ ein Halogenatom darstellt.

15. Verfahren nach dem vorangehenden Anspruch, **dadurch gekennzeichnet, dass** R¹ einen Phenylrest darstellt, R² einen Methylrest darstellt und R³ ein Bromatom darstellt.

## Claims

1. Process for preparing the hexahydropyridazine-3-carboxylic acid derivatives of formula (I) 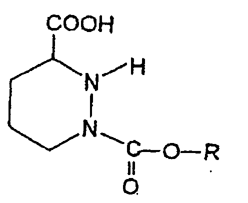 in which R represents a saturated or unsaturated, substituted or unsubstituted alkyl radical, a substituted or unsubstituted aralkyl radical or a substituted or unsubstituted aryl radical, **characterized in that** a compound of formula (II) 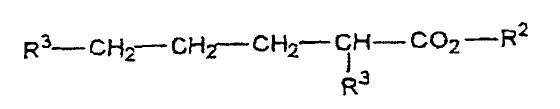 in which R² represents a substituted or unsubstituted alkyl radical, and
R³ represents a halogen atom or a nucleofugal organic group,
is reacted with a compound of formula (III) 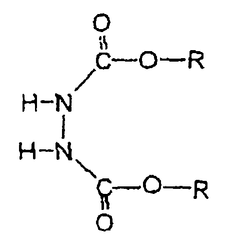 in which R has the above meaning,
in the presence of a base with a pK of greater than or equal to 8.5, in an organic solvent chosen from ketones, to obtain the tetrahydro-1,2,3-pyridazinetricarboxylate intermediate compound of formula (IV) 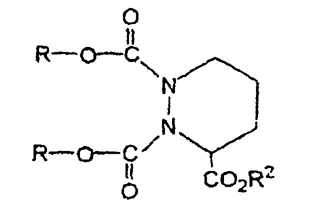 in which R and R² have the above meanings, which is not isolated and which is treated with a basic aqueous medium, to obtain the hexahydropyridazine-3-carboxylic acid derivative of formula (I).

2. Process according to Claim 1, **characterized in that** the organic solvent is chosen from acetone, methyl ethyl ketone, methyl isobutyl ketone, methyl tert-butyl ketone and diisopropyl ketone, and mixtures thereof.

3. Process according to Claim 1 or 2, **characterized in that** the base used in the first reaction is chosen from the group consisting of alkali metal carbonates and tertiary amines.

4. Process according to any one of the preceding claims, **characterized in that** the solvent is acetone.

5. Process according to any one of the preceding claims, **characterized in that** the base used in the first reaction is potassium carbonate.

6. Process according to any one of the preceding claims, **characterized in that** the base used for the second reaction is chosen from alkali metal hydroxides and alkali metal or alkaline-earth metal alkoxides.

7. Process according to the preceding claim, **characterized in that** the alkali metal hydroxides are used in aqueous solution.

8. Process according to Claim 6 or 7, **characterized in that** the mineral base is sodium hydroxide or potassium hydroxide.

9. Process according to any one of the preceding claims, **characterized in that**, for the second reaction, the temperature is between 25°C and 55°C and the volume of water is between 1 and 10 litres per kilogram of compound of formula (III).

10. Process according to the preceding claim, **characterized in that** the reaction is performed by applying different successive temperature stages within the range.

11. Process according to any one of the preceding claims, **characterized in that** the compound of formula (I) is obtained in crystalline form by mixing the reaction medium with a solvent in which the compound of formula (I) is insoluble and which is a diluent for alcohols, and by bringing the pH of the medium to a value of between 0.5 and 2 using an acid.

12. Process according to the preceding claim, **characterized in that** the solvent is chosen from the group consisting of aromatic hydrocarbons, aliphatic hydrocarbons, ethers and acetates.

13. Process according to Claim 11 or 12, **characterized in that** the acid is hydrochloric acid.

14. Process according to any one of the preceding claims, **characterized in that** R represents the radical -CH₂-R¹, R¹ represents the phenyl or naphthyl radical, R² represents a C₁ to C₄ alkyl radical and R³represents a halogen atom.

15. Process according to the preceding claim, **characterized in that** R¹ represents the phenyl radical, R² represents a methyl radical and R³represents a bromine atom.
